# FASCICULE DE BREVET EUROPEEN

(11) **EP 0 887 329 B1**
(45) Date de publication et mention de la délivrance du brevet: **25.05.2005**
(21) Numéro de dépôt: 98401378.9
(22) Date de dépôt: 09.06.1998
(51) Int. Cl.: C07C 19/08, C07C 17/269, C07C 17/087

(54) **Procédé de fabrication d'hydrofluoroalcanes**
Verfahren zur Herstellung von Fluorkohlenwasserstoffen
Process for the preparation of hydrofluoroalkanes

(30) Priorité: 18.06.1997 FR 9707576
(43) Date de publication de la demande: 30.12.1998
(73) Titulaire: ARKEMA, 92800 Puteaux (FR)
(72) Inventeur: Sage, Jean-Marc, 69600 Oullins (FR); Lacroix, Eric, 69480 Amberieux d'Azergues (FR); Bonnet, Philippe, 69007 Lyon (FR); Jorda, Eric, 69005 Lyon (FR)

(56) Documents cités:
- EP-A- 0 036 123
- EP-A- 0 773 206
- US-A- 3 009 966
- US-A- 3 154 592

## Description

La présente invention concerne le domaine des hydrofluoroalcanes, appelés communément HFC ou HFA, et a plus particulièrement pour objet la fabrication du pentafluoroéthane (F125) et du 1,1,1,2,3,3,3-heptafluoropropane (F227ea) à partir du trifluorométhane, connu sous la désignation F23.

L'intérêt pour les hydrofluoroalcanes grandit depuis que l'on suspecte les chlorofluoroalcanes (CFC) de contribuer à l'affaiblissement de la couche d'ozone stratosphérique.

Les CFC tels que le fluorotrichlorométhane (CFC 11), le dichlorodifluorométhane (CFC 12), le 1,1,2-trichloro-1,2,2-trifluoroéthane (CFC 113) et le chloropentafluoroéthane (CFC 115), ont de ce fait été interdits dans tous les pays industrialisés et sont remplacés par des hydrofluoroalcanes tels que le 1,1,1,2-tétrafluoroéthane (F134a), le difluorométhane (F32), le pentafluoroéthane (F125), le 1,1,1,2,3,3,3-heptafluoropropane (F227ea), le 1,1,1,3,3-pentafluoropropane (F245fa) et le 1,1,1,2,2-pentafluorobutane (F365mfc), ainsi que par des hydrochlorofluoroalcanes (HCFC) tels que le chlorodifluorométhane (F22), le 1,1-dichloro-1-fluoroéthane (F141b) et le 1-chloro-1,1-difluoroéthane (F142b). Bien que moins nocifs que les CFC pour la couche d'ozone, les HCFC sont cependant appelés progressivement à disparaître. Il est donc nécessaire de pouvoir fabriquer les produits de type HFC pour remplacer les CFC et les HCFC.

La majorité des procédés actuellement connus pour la synthèse des HFC sont basés sur la fluoration catalytique de composés chlorés à l'aide de fluorure d'hydrogène ou sur l'hydrogénolyse d'un composé chlorofluoré ou encore sur la pyrolyse d'un HCFC en présence d'HFC. Il est évident que tous ces procédés engageant un produit chloré et coproduisant de l'acide chlorhydrique, donnent en impuretés des produits chlorés de type HCFC ou CFC peu désirables en raison de leurs effets sur la couche d'ozone. La teneur des HFC ainsi fabriqués en impuretés chlorées de type CFC ou HCFC doit être aussi faible que possible et est donc un facteur important à prendre en compte.

En raison des applications diverses de ces produits (réfrigération, climatisation, expansion de mousses, solvant ou extinction de feux), il peut s'avérer nécessaire de disposer de plusieurs HFC possédant différentes propriétés physiques et chimiques ou de mélanges d'HFC satisfaisant plus précisément à certaines applications.

Disposer d'un procédé permettant de fabriquer plusieurs HFC de propriétés physiques différentes à partir d'un même produit de départ, sans conduire à la formation de sous-produits et impuretés chlorés de type HCFC ou CFC ou encore d'acide chlorhydrique, serait particulièrement intéressant dans ce domaine, notamment pour fabriquer le pentafluoroéthane (F125) et le 1,1,1,2,3,3,3-heptafluoropropane (F227ea).

Parmi les nombreux procédés de synthèse du F125 à partir d'un dérivé chloré ou chlorofluoré, on peut citer :
- ceux relatifs à la fluoration du 1,1,1-trifluoro-2,2-dichloroéthane (F123) par le fluorure d'hydrogène en phase gaz en présence d'un catalyseur contenant du chrome déposé sur un support charbon (brevet EP 456 552) ou en présence d'un catalyseur contenant du chrome sur un support de type alumine fluorée (brevet EP 349 298),
- la dismutation d'un dérivé HCFC tel que le F124 dont le passage sur un catalyseur de type oxyde de chrome conduit en sortie à un mélange de F125 et de F123 (demande de brevet WO 9202476),
- l'hydrogénolyse du F115 (demande WO 9105752 et brevet EP 0 506 525), un taux de transformation de F115 supérieur à 99 % réclamant des conditions poussées qui conduisent à la formation de F143a (CF₃-CH₃) en quantités importantes.

Ces procédés mettant en jeu des dérivés chlorés du type HCFC ou CFC impliquent en général une purification poussée du F125 obtenu, l'un des problèmes majeurs étant la présence dans le F125 de F115 difficilement séparable par simple distillation et qu'il faut éliminer par des techniques plus complexes, comme décrit dans le brevet FR 2 716 449.

Le F125 peut également être obtenu par fluoration du tétrafluoroéthylène en phase liquide (brevet US 4 258 225) ou gazeuse (brevet EP 0 036 123) en présence de catalyseurs, mais ces procédés nécessitent l'isolement ou le stockage du C₂F₄ ce qui constitue un handicap majeur en raison des dangers d'explosion ou de polymérisation inhérent à ce produit. Selon le brevet RU 2 049 085, la fluoration du C₂F₄ en mélange avec le F124 peut être effectuée en phase gaz avec un catalyseur à base de chrome sur un oxyde d'aluminium : le C₂F₄ et le F124 peuvent être issus de la pyrolyse du F22 (CHCIF₂), mais le F125 ainsi obtenu est contaminé par des HCFC comme le F114 et le F115, ce qui diminue considérablement l'intérêt d'un tel procédé.

Le brevet FR 2 731 701 décrit un procédé de synthèse de F125 à partir d'un mélange de F23 et de F22. Bien que ce procédé permette de produire d'autres HFC que le F125, il nécessite l'utilisation d'un dérivé de départ chloré le F22 ; d'autre part, la présence de l'HCl formé par la réaction est également peu désirable en raison des problèmes de corrosion et des sous-produits chlorés qu'il peut engendrer dans le milieu réactionnel.

Le brevet US 3 009 966 décrit un procédé de fabrication d'oléfines perfluorées comprenant une étape de pyrolyse du F23 et signale qu'il se forme aussi du F125 et du F227ea en quantités très minoritaires.

Parmi les différents procédés de synthèse du F227ea on peut mentionner l'hydrogénolyse du 2-chloroheptafluoropropane sur des catalyseurs métalliques (brevet EP 539 989) et la fluoration par HF du perfluoropropène en présence d'un catalyseur antimonié (demande de brevet WO 9602483).

Il a maintenant été trouvé qu'à partir du trifluorométhane (F23), il est possible de préparer avec de bons rendements le pentafluoroéthane (F125), le 1,1,1,2,3,3,3-heptafluoropropane (F227ea) ou un mélange de ces composés, par un procédé qui ne met en jeu aucun produit ou sous-produit chloré et fournit donc des F125 et F227ea exempts des impuretés de type CFC ou HCFC usuellement présentes dans les produits obtenus par les procédés basés sur l'emploi de matières premières chlorées. Ce procédé permet en outre de coproduire de l'acide fluorhydrique exempt de toute trace d'acide chlorhydrique.

L'invention a donc pour objet un procédé de fabrication de pentafluoroéthane (F125) et/ou de 1,1,1,2,3,3,3-heptafluoropropane (F227ea) caractérisé en ce qu'il comprend :
(a) une étape consistant à soumettre en phase gazeuse un courant de trifluorométhane (F23) à une pyrolyse à une température supérieure à 700°C, et
(b) une étape consistant à mettre le mélange de gaz issus de l'étape de pyrolyse, sans séparation, en contact avec un catalyseur de fluoration.

Dans l'étape (a) du procédé selon l'invention, la pyrolyse du F23 conduit à la formation de tétrafluoroéthylène et/ou de perfluoropropène et à la coproduction de fluorure d'hydrogène.

Dans l'étape (b) menée en présence d'un catalyseur de fluoration, une partie de l'HF co-produit à l'étape (a) s'additionne sur le tétrafluoroéthylène et/ou le perfluoropropène pour former le F125 et/ou le F227ea.

Comme le montre le schéma réactionnel suivant: 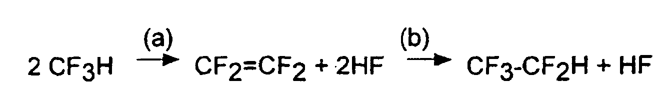 et/ou 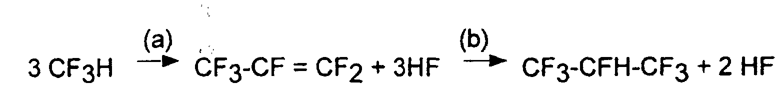 l'HF formé lors de l'étape (a) n'est que partiellement consommé lors de l'étape (b). L'excédent peut ainsi facilement être récupéré pour être valorisé dans d'autres fabrications. D'autre part, l'absence d'acide chlorhydrique dans le milieu réactionnel évite les problèmes de corrosion induits par la présence d'acide chlorhydrique à haute température.

L'étape (a) peut être réalisée sous vide ou sous pression. La pyrolyse peut ainsi être menée sous une pression absolue allant de 10 mbars jusqu'à 10 bars, une pression trop forte entraînant des pertes importantes de sélectivité en produit et une pression trop faible diminuant la productivité du procédé. Le choix de cette pression est également lié à l'orientation désirée vers la production intermédiaire de C₂F₄ ou de C₃F₆ lors de la première étape ou bien la production finale de F125 ou de F227ea à l'issue de la deuxième étape. Une pression basse est favorable à la formation de C₂F₄ et donc de F125 tandis qu'une pression plus élevée favorise la formation de C₃F₆ et donc de F227ea. De préférence, on travaille sous une pression comprise entre 50 mbars et 2 bars, une valeur de pression égale à la pression atmosphérique étant de plus préférée.

L'étape de pyrolyse (a) est menée à une température supérieure à 700°C, mais le choix exact de la température de pyrolyse dépend du temps de contact choisi et des produits désirés en sortie du procédé ; la limite haute de cette température est généralement fixée par les contraintes technologiques. Cette température de pyrolyse est usuellement comprise entre 700 et 1200°C ; elle est de préférence choisie entre 900 et 1100°C et, de façon encore plus préférée, entre 950 et 1050°C.

Le temps de contact tel qu'il est indiqué ci-après est défini par le rapport du volume de la zone chauffée sur le débit gazeux exprimé en volume par unité de temps, ce débit gazeux étant ramené aux conditions de pression et température de l'étape réactionnelle en appliquant la relation des gaz parfaits. Le choix du temps de contact lors de l'étape (a) est évidemment lié aux conditions de température et de pression mises en jeu. Il est généralement compris entre 0,1 milliseconde et 2 secondes, de préférence entre 5 millisecondes et 0,1 seconde et, de façon encore plus préférée, entre 5 et 50 millisecondes. La limite basse de ce temps de contact est en fait dictée par les limites technologiques concernant les températures et les débits gazeux à mettre en oeuvre.

Le réacteur dans lequel est effectué l'étape de pyrolyse (a) est de préférence un réacteur de type tubulaire vide, mais on peut également utiliser un réacteur garni d'un remplissage inerte afin d'augmenter les surfaces d'échange. Bien qu'on puisse introduire dans le réacteur un catalyseur massique ou supporté, l'étape de pyrolyse ne nécessite pas usuellement de catalyseur et est de préférence réalisée en absence de catalyseur.

Le réacteur peut être fait de divers matériaux supportant les conditions de température et de pression et résistant à la corrosion. On peut pour cela utiliser des réacteurs revêtus intérieurement par exemple de platine ; toutefois on préfère les matériaux comme l'Inconel, le nickel ou l'Hastelloy.

Dans le réacteur de pyrolyse, le F23 peut être introduit pur ou en mélange avec un recyclat de différents produits formés au cours du procédé. Ces produits et sous-produits peuvent être le F125, le F227ea, le perfluorocyclobutane (cC₄F₈), le tétrafluoroéthylène, le perfluoropropène, l'hexafluoroéthane (F116) ou encore l'acide fluorhydrique. On peut également introduire un gaz diluant inerte tel que l'azote.

Les gaz issus de l'étape de pyrolyse (a) et contenant comme produits principaux du F23 non réagi, de l'HF et un mélange de perfluoropropène et de tétrafluoroéthylène qui, selon l'orientation choisie, peut contenir majoritairement l'un ou l'autre de ces deux produits sont envoyés dans un réacteur contenant un catalyseur de fluoration et fonctionnant à une température inférieure à 500°C.

Le mélange brut des gaz issus de l'étape de pyrolyse (a) est de préférence envoyé directement au réacteur de fluoration, mais il peut également faire l'objet d'un stockage intermédiaire ou tampon afin de faciliter la conduite du procédé. Au mélange des gaz provenant de l'étape de pyrolyse, on peut ajouter des produits de recyclage et notamment du fluorure d'hydrogène. Pour stabiliser les oléfines perfluorées et éviter leur polymérisation, on peut également ajouter au flux alimentant le réacteur de fluoration un ou plusieurs stabilisants tels que, par exemple, des terpènes comme le limonène ou le mélange connu sous le nom de Dipsol®, les phénols, les amines et certains dérivés aromatiques comme le toluène ou l'alphaméthylstyrène.

La réaction de fluoration peut être menée en phase gazeuse ou en phase liquide.

Lorsque la réaction est menée en phase gazeuse, on utilise généralement un catalyseur à base de fluorures et/ou d'oxydes métalliques comme ceux du chrome, du nickel, du zinc, du magnésium, du calcium ou du fer. Le catalyseur utilisé peut être de nature massique ou supporté sur un substrat qui peut être un fluorure d'alumine, un charbon ou tout autre support résistant à la présence de fluorure d'hydrogène. Les catalyseurs à base de chrome donnent de bons résultats et un des catalyseurs préférés consiste en un catalyseur contenant du chrome déposé sur un support à base d'alumine fluorée.

La température de l'étape de fluoration, menée en phase gazeuse, peut être comprise entre 200 et 500°C ; on préfère cependant opérer à une température comprise entre 200 et 400°C et, plus particulièrement, entre 250 et 350°C. En phase gazeuse, la pression de l'étape de fluoration peut varier entre 10 mbars et 20 bars ; elle est avantageusement comprise entre 100 mbars et 5 bars et plus avantageusement, égale à la pression atmosphérique. Il est évident que, d'un point de vue technologique, on a avantage à choisir des pressions similaires pour les deux étapes du procédé.

Le temps de contact lors de cette étape de fluoration en phase gaz est compris entre 0,1 et 120 secondes ; de préférence on opère avec un temps de contact compris entre 1 et 30 secondes.

On peut également effectuer l'étape de fluoration (b) en phase liquide sous pression. La réaction peut alors être réalisée en présence ou non d'un solvant, notamment en milieu dit HF dans lequel l'acide fluorhydrique sert de milieu solvant.

Comme catalyseur pour la fluoration en phase liquide, on peut mentionner les catalyseurs usuels que sont les dérivés de l'antimoine, du titane, de l'étain, du tantale ou du niobium. La température de fluoration en phase liquide peut varier entre 20 et 150°C.

En sortie de l'étape de fluoration, les gaz contenant principalement du F23, de l'acide fluorhydrique, du F125 et/ou F227ea sont séparés. On préfère opérer dans des conditions ne fournissant pas d'oléfines C₂F₄ ou C₃F₆ résiduelles ; cependant, si ces oléfines sont encore présentes en sortie de l'étape de fluoration, elles peuvent être soit recyclées dans le procédé, soit récupérées pour d'autres usages.

On peut laver les gaz en sortie par une solution aqueuse afin d'éliminer l'acide fluorhydrique puis distiller les gaz afin de récupérer le F125 et/ou le F227ea formé ; le F23 est généralement retourné en début de réaction ainsi que tout ou partie des autres sous-produits ou produits intermédiaires non transformés. On peut également distiller les gaz bruts issus de cette deuxième étape afin de récupérer l'HF sous une forme plus facilement réutilisable industriellement ou récupérer le fluorure d'hydrogène par lavage avec de l'acide sulfurique.

Les exemples suivants illustrent l'invention.

### EXEMPLE 1

### (a) Préparation du catalyseur de fluoration

Dans un réacteur en Inconel de 1,5 litre et de 8 cm de diamètre interne, chauffé au moyen d'un dispositif à bain de sable fluidisé, on charge 1 litre d'une alumine imprégnée à 6 % en chrome au moyen d'une solution d'oxyde de chrome CrO₃ dans le méthanol.

L'alumine employée est une alumine commerciale présentant les caractéristiques suivantes :
- forme : billes de 1-2 mm
- surface BET : 223 m²/g
- volume poreux : 1,2 cm³/g (pour les rayons de pores compris entre 4 nm et 63 µm)

Le catalyseur est séché une nuit sous balayage d'azote à 200°C, puis à cette température et en présence d'azote, on introduit 240 g de fluorure d'hydrogène à un débit de 18 g/h. La température est ensuite portée à 350°C et l'on poursuit l'introduction d'HF au même débit pendant encore12 heures. On obtient ainsi le catalyseur de fluoration utilisé pour la deuxième étape du procédé.

### (b) Pyrolyse du trifluorométhane

Le trifluorométhane (F23) préchauffé à 170°C est introduit dans un réacteur constitué d'un tube vide en nickel, d'une longueur d'environ 50 cm, de diamètre interne 4 mm et de diamètre externe 6 mm. Ce tube est chauffé par un four; la longueur réellement chauffée à une température proche de la température de consigne (température comprise entre la température de consigne et cette même température diminuée de 20°C) a été évaluée expérimentalement à 20 cm par introduction d'une sonde de température dans ce tube et en faisant passer de l'azote, cette sonde de température étant retirée lors des essais. Cette longueur est prise en compte pour évaluer le temps de contact lors de cette première étape. La température de consigne (T1) pour cet exemple est de 1000°C et le temps de contact (tc1) est de 32 millisecondes.

### (c) Fluoration du produit de pyrolyse

Le réacteur dans lequel est effectuée cette deuxième étape du procédé est le réacteur décrit au paragraphe (a) et est chargé avec le catalyseur préparé au paragraphe (a). Les gaz issus de l'étape de pyrolyse décrite au paragraphe (b) sont directement envoyés vers ce réacteur de fluoration. La température de cette deuxième étape (T2) est fixée à 300°C ; la pression est égale à la pression atmosphérique et le temps de contact (tc2), calculé par rapport au volume de catalyseur, est de 29 secondes. A la sortie du deuxième réacteur, les gaz sont lavés à l'eau puis séchés sur du chlorure de calcium.

Les résultats obtenus sont reportés dans le tableau suivant.

### EXEMPLE 2

On opère comme à l'exemple 1, mais en changeant le débit de F23 admis, c'est-à-dire en modifiant les temps de contact des deux étapes du procédé. Les résultats sont reportés dans le tableau suivant.

### EXEMPLE 3

On opère de manière similaire aux exemples précédents mais en utilisant un réacteur de pyrolyse (première étape) de diamètre interne 2 mm et de diamètre externe 4 mm, la zone de chauffe étant égale à 20 cm. D'autre part, on opère avec un volume de catalyseur plus faible lors de la deuxième étape afin d'avoir un temps de contact de 15 secondes. Les résultats obtenus sont reportés dans le tableau suivant.

| **Exemple** | **T1 °C** | **tc1 secondes** | **T2 °C** | **tc2 secondes** | **F23** | **F125** | **F227ea** | **cC**_{**4**}**F**_{**8**} | **F116** | **C**_{**4**}**F**_{**9**}**H** | **autres** |
|---|---|---|---|---|---|---|---|---|---|---|---|
| | | | | | **% molaires (analyse CPV)** | | | | | | |
| 1 | 1000 | 0,032 | 300 | 29 | 78,4 | 15,1 | 4,04 | 0,14 | 1,15 | 0,16 | 1,01 |
| 2 | 1000 | 0,025 | 300 | 22 | 84,8 | 11,4 | 2,0 | 0,10 | 0,97 | 0,08 | 0,65 |
| 3 | 1000 | 0,010 | 300 | 15 | 86,2 | 12,0 | 0,99 | 0,05 | 0,32 | 0,02 | 0,42 |

### EXEMPLE 4

On a opéré dans les mêmes conditions qu'à l'exemple 3. Après plus de 100 heures de marche, on n'a noté aucun signe de désactivation du catalyseur de fluoration et la répartition des produits est restée inchangée.

### EXEMPLE 5

L'appareillage utilisé est constitué d'un autoclave de 1l en inox 316L à double enveloppe, surmonté d'un condenseur simple également à double enveloppe et d'une vanne de régulation de pression.

Dans cet autoclave plongé dans l'azote liquide, sont successivement chargés 502 g d'HF (25.1 moles) et 71.7 g de TaCl₅ (0.2 mole). Puis la température de l'autoclave est ramenée à température ambiante. L'autoclave est alors plongé dans un bain d'huile de façon à obtenir une température d'environ 100°C dans le milieu réactionnel, tandis que la température en tête du condenseur est maintenue à 20°C et l'ensemble est régulé à une pression de 10 bars.

Lorsque la température de 100°C est atteinte, on alimente en phase gaz du C₂F₄ à un débit de 14,9 g/h (0.3 mole/h). Les produits les plus volatils, évacués en continu en tête du condenseur, passent dans un barboteur à eau puis dans un sécheur, avant d'être recueillis dans un récipient en inox refroidi à l'azote liquide.

Après 2h de réaction, l'autoclave est mis en refroidissement par circulation d'eau dans le bain d'huile. Après le retour à la température ambiante, l'autoclave est dégazé et les produits de la réaction sont lavés, séchés et recueillis comme précédemment. Les phases gaz et les phases liquide ainsi recueillies sont analysées ainsi que la phase liquide restant éventuellement dans l'autoclave après le dégazage.

La conversion (exprimée par rapport à 100 moles de C₂F₄ introduit) s'élève à 12.9 % avec une sélectivité (exprimée par rapport au nombre de moles de C₂F₄ consommé par la réaction) en F125 de 99.9 %.

### EXEMPLE 6

On répète l'exemple 5 en chargeant dans l'autoclave plongé dans l'azote liquide, successivement 482 g d'HF (24,1 moles) et 50,6 g de NbCl₅ (0,19 mole) et en alimentant le C₂F₄ en phase gaz avec un débit de 15,1 g/h (0,3 moles).

Après 2h de réaction, l'analyse des phases gaz et liquide montre que la conversion du C₂F₄ introduit s'élève à 11 % avec une sélectivité en F125 de 99,9 %.

Les exemples 5 et 6 montrent que le mélange de gaz issu de la réaction de pyrolyse du 23 selon l'étape (a) du procédé selon invention peut être soumis à une réaction de fluoration en phase liquide catalysée, pour donner du F125.

## Revendications

1. Procédé de fabrication de pentafluoroéthane et/ou de 1,1,1,2,3,3,3-heptafluoropropane, **caractérisé en ce qu'**il comprend une étape consistant à soumettre en phase gazeuse un courant de trifluorométhane à une pyrolyse à une température supérieure à 700°C et une étape consistant à mettre le mélange de gaz issu de l'étape de pyrolyse, sans séparation, en contact avec un catalyseur de fluoration

2. Procédé selon la revendication 1 dans lequel l'étape de pyrolyse est menée à une température allant de 700°C à 1200°C, de préférence à une température comprise entre 900°C et 1100°C et, plus particulièrement, à une température comprise entre 950°C et 1050°C.

3. Procédé selon la revendication 1 ou 2, dans lequel l'étape de pyrolyse est menée sous une pression allant de 10 mbars à 10 bars, de préférence sous une pression comprise entre 50 mbars et 2 bars et, plus particulièrement, à la pression atmosphérique.

4. Procédé selon la revendication 2 ou 3, dans lequel le temps de contact est compris entre 0,1 milliseconde et 2 secondes, de préférence entre 5 millisecondes et 0,1 seconde et, plus particulièrement, entre 5 et 50 millisecondes.

5. Procédé selon l'une des revendications 1 à 4, dans lequel le réacteur de pyrolyse est un réacteur tubulaire vide.

6. Procédé selon l'une des revendications 1 à 5, dans lequel le trifluorométhane alimenté au réacteur de pyrolyse est pur, dilué par un gaz inerte et/ou mélangé à des produits recyclés.

7. Procédé selon la revendication 6, dans lequel les produits recyclés comprennent du pentafluoroéthane, du 1,1,1,2,3,3,3-heptafluoropropane, du perfluorocyclobutane, du tétrafluoroéthylène, du perfluoropropène, de l'hexafluoroéthane et/ou du fluorure d'hydrogène.

8. Procédé selon l'une des revendications 1 à 7, dans lequel l'étape de fluoration est menée en phase gazeuse à une température allant de 20°C à 500°C, de préférence à une température comprise entre 200°C et 400°C et, plus particulièrement, à une température comprise entre 250°C et 350°C.

9. Procédé selon la revendication 8, dans lequel l'étape de fluoration est menée sous une pression allant de 10 mbars à 20 bars, de préférence sous une pression comprise entre 100 mbars et 5 bars et, plus particulièrement, à la pression atmosphérique.

10. Procédé selon la revendication 8 ou 9, dans lequel le temps de contact est compris entre 0,1 et 120 secondes, de préférence entre 1 et 30 secondes.

11. Procédé selon l'une des revendications 8 à 10, dans lequel le catalyseur de fluoration est à base de chrome.

12. Procédé selon l'une des revendications 1 à 7, dans lequel l'étape de fluoration est menée en phase liquide sous pression.

13. Procédé selon la revendication 12, dans lequel le catalyseur de fluoration est à base d'antimoine, de titane ou d'étain.

14. Procédé selon la revendication 12 ou 13, dans lequel la température est comprise entre 20°C et 150°C.

15. Procédé selon l'une des revendications 12 à 14, dans lequel la fluoration est effectuée en milieu solvant, de préférence en milieu HF.

16. Procédé selon l'une des revendications 8 à 15, dans lequel on ajoute au flux gazeux issu de l'étape de pyrolyse des sous-produits du procédé.

17. Procédé selon l'une des revendications 8 à 16, dans lequel on ajoute un stabilisant au flux gazeux issus de l'étape de pyrolyse.

## Patentansprüche

1. Verfahren zur Herstellung von Pentafluorethan und/oder 1,1,1,2,3,3,3-Heptafluorpropan, **gekennzeichnet durch** einen Schritt, bei dem man einen Strom von Trifluormethan in der Gasphase bei einer Temperatur von mehr als 700°C pyrolysiert, und einen Schritt, bei dem man das bei dem Pyrolyseschritt anfallende Gasgemisch ohne Trennung mit einem Fluorierungskatalysator in Kontakt bringt.

2. Verfahren nach Anspruch 1, bei dem man den Pyrolyseschritt bei einer Temperatur von 700°C bis 1200°C, vorzugsweise bei einer Temperatur zwischen 900°C und 1100°C und insbesondere bei einer Temperatur zwischen 950°C und 1050°C durchführt.

3. Verfahren nach Anspruch 1 oder 2, bei dem man den Pyrolyseschritt bei einem Druck von 10 mbar bis 10 bar, vorzugsweise unter einem Druck zwischen 50 mbar und 2 bar und insbesondere unter Normaldruck durchführt.

4. Verfahren nach Anspruch 2 oder 3, bei dem die Kontaktzeit zwischen 0,1 Millisekunden und 2 Sekunden, vorzugsweise zwischen 5 Millisekunden und 0,1 Sekunden und insbesondere zwischen 5 und 50 Millisekunden liegt.

5. Verfahren nach einem der Ansprüche 1 bis 4, bei dem es sich bei dem Pyrolysereaktor um einen leeren Rohrreaktor handelt.

6. Verfahren nach einem der Ansprüche 1 bis 5, **dadurch gekennzeichnet, daß** das in den Pyrolysereaktor eingespeiste Trifluormethan rein und mit einem Inertgas verdünnt und/oder mit rezyklierten Produkten vermischt ist.

7. Verfahren nach Anspruch 6, bei dem die rezyklierten Produkte Pentafluorethan, 1,1,1,2,3,3,3-Heptafluorpropan, Perfluorcyclobutan, Tetrafluorethylen, Perfluorpropen, Hexafluorethan und/oder Fluorwasserstoff umfassen.

8. Verfahren nach einem der Ansprüche 1 bis 7, bei dem man den Fluorierungsschritt in der Gasphase bei einer Temperatur von 20°C bis 500°C, vorzugsweise bei einer Temperatur zwischen 200°C und 400°C und insbesondere bei einer Temperatur zwischen 250°C und 350°C durchführt.

9. Verfahren nach Anspruch 8, bei dem man den Fluorierungsschritt bei einem Druck von 10 mbar bis 20 bar, vorzugsweise unter einem Druck zwischen 100 mbar und 5 bar und insbesondere unter Normaldruck durchführt.

10. Verfahren nach Anspruch 8 oder 9, bei dem die Kontaktzeit zwischen 0,1 und 120 Sekunden und vorzugsweise zwischen 1 und 30 Sekunden liegt.

11. Verfahren nach einem der Ansprüche 8 bis 10, bei dem der Fluorierungskatalysator auf Chrom basiert.

12. Verfahren nach einem der Ansprüche 1 bis 7, bei dem man den Fluorierungsschritt in der Flüssigphase unter Druck durchführt.

13. Verfahren nach Anspruch 12, bei dem der Fluorierungskatalysator auf Antimon, Titan oder Zinn basiert.

14. Verfahren nach Anspruch 12 oder 13, bei dem die Temperatur zwischen 20 und 150°C liegt.

15. Verfahren nach einem der Ansprüche 12 bis 14, bei dem man den Fluorierungsschritt in einem Lösungsmittelmedium, vorzugsweise in HF-Medium, durchführt.

16. Verfahren nach einem der Ansprüche 8 bis 15, bei dem man dem bei dem Pyrolyseschritt anfallenden Gasstrom Nebenprodukte des Verfahrens zusetzt.

17. Verfahren nach einem der Ansprüche 8 bis 16, bei dem man dem bei dem Pyrolyseschritt anfallenden Gasstrom ein Stabilisierungsmittel zusetzt.

## Claims

1. Process for the manufacture of pentafluoroethane and/or of 1,1,1,2,3,3,3-heptafluoropropane, **characterized in that** it comprises a stage which consists in subjecting, in the gas phase, a stream of trifluoromethane to pyrolysis at a temperature of greater than 700°C and a stage which consists in bringing the mixture of gases which results from the pyrolysis stage, without separation, into contact with a fluorination catalyst.

2. Process according to Claim 1, in which the pyrolysis stage is carried out at a temperature ranging from 700°C to 1200°C, preferably at a temperature of between 900°C and 1100°C and more particularly at a temperature of between 950°C and 1050°C.

3. Process according to Claim 1 or 2, in which the pyrolysis stage is carried out under a pressure ranging from 10 mbar to 10 bar, preferably under a pressure of between 50 mbar and 2 bar and more particularly at atmospheric pressure.

4. Process according to Claim 2 or 3, in which the contact time is between 0.1 millisecond and 2 seconds, preferably between 5 milliseconds and 0.1 second and more particularly between 5 and 50 milliseconds.

5. Process according to one of Claims 1 to 4, in which the pyrolysis reactor is an empty tubular reactor.

6. Process according to one of Claims 1 to 5, in which the trifluoromethane supplied to the pyrolysis reactor is pure, diluted with an inert gas and/or mixed with recycled products.

7. Process according to Claim 6, in which the recycled products comprise pentafluoroethane, 1,1,1,2,3,3,3-heptafluoropropane, perfluorocyclobutane, tetrafluoroethylene, perfluoropropene, hexafluoroethane and/or hydrogen fluoride.

8. Process according to one of Claims 1 to 7, in which the fluorination stage is carried out in the gas phase at a temperature ranging from 20°C to 500°C, preferably at a temperature of between 200°C and 400°C and more particularly at a temperature of between 250°C and 350°C.

9. Process according to Claim 8, in which the fluorination stage is carried out under a pressure ranging from 10 mbar to 20 bar, preferably under a pressure of between 100 mbar and 5 bar and more particularly at atmospheric pressure.

10. Process according to Claim 8 or 9, in which the contact time is between 0.1 and 120 seconds, preferably between 1 and 30 seconds.

11. Process according to one of Claims 8 to 10, in which the fluorination catalyst is based on chromium.

12. Process according to one of Claims 1 to 7, in which the fluorination stage is carried out in the liquid phase under pressure.

13. Process according to Claim 12, in which the fluorination catalyst is based on antimony, on titanium or on tin.

14. Process according to Claim 12 or 13, in which the temperature is between 20°C and 150°C.

15. Process according to one of Claims 12 to 14, in which the fluorination is carried out in solvent medium, preferably in HF medium.

16. Process according to one of Claims 8 to 15, in which byproducts of the process are added to the gas flow resulting from the pyrolysis stage.

17. Process according to one of Claims 8 to 16, in which a stabilizing agent is added to the gas flow resulting from the pyrolysis stage.
